# EUROPEAN PATENT SPECIFICATION

(11) **EP 1 094 849 B1**
(45) Date of publication and mention of the grant of the patent: **17.05.2006**
(21) Application number: 99935483.0
(22) Date of filing: 09.07.1999
(51) Int. Cl.: A61L 15/32, A61L 15/44, A61L 31/00, A61L 33/00, A61K 38/39, A61K 31/721, A61P 15/02

(54) **METHODS FOR INCREASING VASCULARIZATION AND PROMOTING WOUND HEALING**
METHODEN ZUR VERBESSERUNG DER VASKULARISIERUNG UND FÖRDERUNG DER WUNDHEILUNG
PROCEDES PERMETTANT D'AUGMENTER LA VASCULARISATION ET DE FAVORISER LA GUERISON DES BLESSURES

(30) Priority: 10.07.1998 US 113437; 22.06.1999 US 337959
(43) Date of publication of application: 02.05.2001
(73) Proprietor: Encelle, Inc., Greenville, NC 27858 (US)
(72) Inventor: USALA, Anton-Lewis, Winterville, NC 27890 (US)
(74) Representative: Sexton, Jane Helen
(86) International application number: PCT/US1999/015614
(87) International publication number: WO 2000/002596

(56) References cited:
- EP-A- 0 526 756
- EP-A- 0 564 786
- WO-A-97/20569
- US-A- 4 950 483
- US-A- 5 645 591

## Description

### FIELD OF INVENTION

The invention is drawn to the use of gelatin and dextran in the manufacture of a matrix for use in wound healing.

### BACKGROUND OF THE INVENTION

Blood vessels are assembled by two processes known as vasculogenesis and angiogenesis. In vasculogenesis, a primitive vascular network is established during embryonic development from endothelial cell precursors called angioblasts. Angiogenesis involves preexisting vessels sending out capillary buds or sprouts to produce new vessels. Angiogenesis is an important process critical to chronic inflammation and fibrosis, to tumor cell growth, and to the formation of collateral circulation. Angiogenesis is involved in the normal process of tissue repair.

Tissue destruction, with damage to both parenchymal cells and stromal framework, occurs in inflammation. Repair to the tissue cannot be accomplished solely by regeneration of parenchymal ceils, even in organs whose cells are able to regenerate. Attempts at repairing tissue damage occur by replacement of non-regenerated cells by connective tissue, which in time produces fibrosis and scarring.

After inflammation, repair of the tissue immediately begins. Fibroblasts and vascular endothelial cells began proliferating to form granulation tissue. Granulation tissue is characterized by the formation of new small blood vessels and the proliferation of fibroblasts. The new vessels are leaky and allow the passage of proteins and red blood cells into the extravascular space.

The inflammatory response is closely intertwined with the process of repair. Inflammation serves to destroy, dilute, or wall off the injurious agent. In turn, inflammation sets into motion a series of events that heal and reconstitute the damaged tissue. While repair begins during the early phases of inflammation, it reaches completion only after the injurious influence has been neutralized. During repair, the injured tissue is replaced by regeneration of native parenchymal cells, by filling of the defect with fibroblastic tissue, commonly known as scarring.

The inflammatory response occurs in the vascularized connective tissue. Circulating cells such as neutrophils, monocytes, eosinophils, lymphocytes, basophils, and platelets are involved. Connective tissue cells are the mast cells, which surround blood vessels, the connective tissue fibroblasts, and occasional resident macrophages and lymphocytes.

Progress has been made in transplant technology. New strategies on the horizon include the creation of man-made tissues or organs. However, the transplanted tissue or organ requires a blood supply. Thus, methods are needed for promoting vascularization in sites of interest.

WO 97/20569 describes hydrogel matrix formulations comprising gelatin and a nitric oxide inhibitor for use in stimulating vascularization.

EP-A-0 564 786 describes a method for processing and preserving collagen-based tissues for transplantation.

US-A-5 645 591 describes a synthetic bone matrix capable of inducing the formation of bone.

EP-A-0 526 756 describes a composition for revitalising scar tissue. The composition includes a bioactive substance, such as a growth factor, immobilized in a matrix for controlled release.

US-A-4 950 483 describes a collagen wound healing matrix, the matrix includes collagen wherein the collagen is not chemically cross-linked by the addition of aldehyde or other chemical additives. Growth factors or other bioactive agents and/or heparin may be added to the collagen matrix.

### SUMMARY OF THE INVENTION

The present invention provides the use of a mixture of gelatin and dextran in the manufacture of a matrix for the treatment of a wound. The matrix is useful in wound healing.

### BRIEF DESCRIPTION OF THE DRAWINGS

Figure 1 shows blood Vessel formation around a device 6 weeks post implant;
Figure 2 shows a vascularized device sheath 16 weeks post implant in a diabetic dog;
Figure 3 provides a graph indicating capsule thickness after 21 and 50 days of implantation; and
Figure 4 provides a graph indicating vascular density after 21 and 50 days of implantation.

### DETAILED DESCRIPTION OF THE INVENTION

The matrix comprising a mixture of gelatin and dextran is useful for promoting vascularization. The matrix has been previously described in U.S. Application Serial No. 09/113,437 and U.S. Patent No. 5,824,331. The matrix is able to immobilize water at appropriate storage temperature and provide binding sites for cells that stimulate growth in terminal cell types, such as beta cells. Dextran is useful as a nitric oxide scavenger.

The matrix stimulates local blood vessel growth within a thin fibrous capsule or sheet. While the invention is not bound by any mechanism of action, it is hypothesized that the matrix collagen fragments serve as both a scaffolding and stimulus for fibroblasts and new, physiologic blood vessel expansion, without stimulating immune cell response. Upon breaking the intimal basement membrane of any tissue, polar amino acid sequences are exposed. For example, injection into a muscle with a needle will provide this tearing. The matrix contains denatured collagen fragments loosely bound to dextran, which bind to the exposed polar surface of the basement membrane. Highly polar amino acid additives may be included in the matrix which aid in binding of the collage fragments to the polar surface of the membrane. Within a few hours, the aqueous portion of the matrix is absorbed by the surrounding tissue, leaving only the peptide fragments bound to the exposed polar surfaces. The nitric oxide inhibitors and scavengers present in the matrix inhibit the attraction and activation of immune cells to the area. The denatured connective tissue monomers that are co-polymerized with the dextran component of the matrix provide scaffolding required for endothelial proliferation.

The connective tissue fragments resemble immature collagen in that they are not cross-linked in the large triple standard helix found in mature collagen. *In utero,* single-stranded collagen monomers are laid down first, then cross-linked with other monomers to form mature collagen. This process is followed by cellular binding and differentiation, as well as new blood vessel supply. Because collagen sequences are conserved in mammalian species, it is believed that the matrix collagen fragments serve both a scaffolding and as a stimulus for fibroblasts and new physiologic blood vessel expansion, without stimulating immune cell response.

The matrix of the present invention is a combination of a gelatin component and a liquid composition. The gelatin acts as a substrate for cellular attachment. The preferred gelatin component is denatured collagen. Denatured collagen contains polar and non-polar amino acids that readily form a gel based on amine, carboxyl group, hydroxyl group, and sulfhydryl group interactions. The matrix is designed to be in a free flowing or liquid phase at host body temperature in order to provide maximum diffusion across the membrane *in vivo.* The matrix remains in solid phase at the lower storage temperatures, such as 4°C.

Boiling or otherwise treating intact collagen to form denatured collagen breaks covalent chemical bonds and increases the number of heat sensitive hydrogen bonds and dipole moment attractions. By replacing the covalent chemical bonds with temperature sensitive bonds and attractions, the desired cells can be embedded in a solid matrix formulation at colder temperatures for sustained storage. Boiling or otherwise treating intact collagen breaks the tightly coiled helical tropocollagen subunits and causes the subunits to open up into separate peptide chains. These uncoiled strands provide multiple binding areas for cells to attach.

The gelatin is present at a concentration of about 0.01 to about 40 mM, preferably about 0.05 to about 30 mM, most preferably about 1 to 5 mM. Advantageously, the gelatin concentration is approximately 1.6 mM. The above concentrations provide a solid phase at storage temperature and a liquid phase at transplant or injection temperature.

The gelatin component of the matrix of the present invention is mixed with a liquid composition. The liquid composition is preferably based upon a standard culture medium, such as Medium 199. supplemented with additives and additional amounts of some medium components, such as supplemental amounts of polar amino acids as described above.

The matrix of the present invention includes dextran, a nitric oxide scavenger. A further scavenger may be included. For example, L-cysteine acts as a nitric oxide scavenger and appears to obscure immune recognition sites by binding or docking to the surface of the cells. L-cysteine also provides disulfide linkages which increases the matrix's resistance to force and further protects the cells contained therein. Nitric oxide (NO) is a pleiotropic mediator of inflammation. NO plays an important role in vascular function during inflammatory responses. NO is a soluble gas produced by endothelial cells, macrophages, and specific neurons in the brain. NO is active in inducing the inflammatory response.

The final concentration of L-cysteine is about 5 to about 5,000 µM, preferably about 10 to about 800 µM, most preferably about 100 to about 800 µM. In one embodiment, the final concentration is about 20 µM.

The matrix of the present invention preferably comprises a nitric oxide inhibitor. For example, aminoguanidine is an L-arginine analogue and acts as a nitric oxide inhibitor. Other L-arginine analogues that act as nitric oxide inhibitors could also be used in the present invention. The final concentration of aminoguanidine is about 5 to about 500 µM, preferably about 10 to about 100 µM, most preferably about 15 to about 25 µM. In one embodiment, the final concentration is about 20 µM.

In order to increase cell binding, intact collagen may be added in small amounts to provide an additional binding network for the cells contained in the matrix. The final concentration of intact collagen is from about 0 to about 5 mM,
preferably 0 to about 2 mM, most preferably about 0.05 to about 0.5 mM. In one embodiment, the concentration of intact collagen is about 0.11 mM.

The matrix of the present invention may optionally include a divalent chelator which increases the rigidity of the matrix by removing inhibition of -NH₂ to -COOH hydrogen bonding. The divalent chelator also protects against microbial contamination of the matrix. A preferred divalent chelator is EDTA. The concentration range for the chelator is about 0 to about 10 mM, preferably 1 to about 8 mM, most preferably about 2 to about 6 mM. In a preferred embodiment, EDTA is present at a concentration of about 4 mM. Conventional antibiotics can also be added to further protect against microbial contamination.

While the matrix of the invention does not require the presence of sera, albumin or other nutrient sources may be added to the matrix if desired. Preferably, the albumin used is of the same species as the cells contained within the matrix. As described above, use of the same species albumin promotes increased robustness in the cells contained in the matrix. The concentration of albumin is about 0 to about 2% by volume, preferably 0 to about 0.5% by volume, most preferably about 0 to about 0.1 % by volume. In a preferred embodiment, the concentration of albumin is about 0.05% by volume.

The matrix may contain an effective amount of polar amino acids therein. The polar amino acids may be selected from the group consisting of arginine, lysine, histidine, glutamic acid, and aspartic acid, or other amino acids or other polar chemicals. An effective amount is the amount necessary to increase the rigidity of the matrix and further enhance binding of the collagen fragment to the polar surface of the basement membrane. In one embodiment, the concentration of polar amino acids is raised to a final concentration of between about 3 to about 150 mM, preferably about 10 to about 65 mM, and more preferably about 15 to about 40 mM.

Advantageously, the added polar amino acids comprise L-glutamic acid, L-lysine, and arginine. The final concentration of L-glutamic acid is about 2 to about 60 mM, preferably about 5 to about 40 mM, most preferably about 10 to about 20 mM. In one embodiment, the concentration of L-glutamic acid is about 15 mM. The final concentration of L-lysine is about 0.5 to about 30 mM, preferably about 1 to about 15 mM, most preferably about 1 to about 10 mM. In one embodiment, the concentration of L-lysine is about 5.0 mM. The final concentration of arginine is about 1 to about 40 mM, preferably about 1 to about 30, most preferably about 5 to about 15 mM. In one embodiment, the final concentration of arginine is about 10 mM.

For long term storage of cells, an effective amount of cryoprotectant may be added that allows the matrix to be stored at lower temperatures without cellular damage. Preferably, the cryoprotectant is metabolically stable and capable of creating an inert cushion to prevent thermal expansion and contraction of cells. A preferred cryoprotectant is sulfated dextran. The cryoprotectant is present at a concentration of about 0 to about 2 mM, preferably 0 to about 1 mM, most preferably about 0 to about 0.1 mM. In one embodiment, the cryoprotectant is present in a concentration of about 0.086 mM. Dextran is also useful as a nitric oxide scavenger.

Table 1 below lists particularly preferred key components of the matrix of the present invention along with suitable concentrations as well as preferred concentrations for each component.

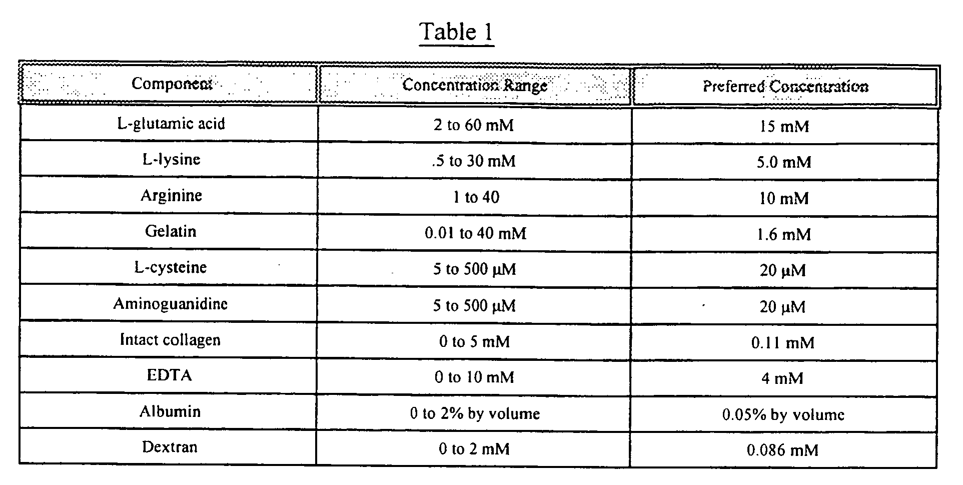

The matrix may be used to stimulate or enhance vascularization in a mammal at an anatomic site without immune cell stimulation at the site, resulting in long term functional vascularity. That is, after insertion of the matrix in a mammal, vascularization is stimulated in tissue surrounding the matrix. "Anatomic site" is a predetermined site in a mammal where vascularization is needed.

Anatomic sites include sites of disease in an organism such as sites of chronic inflammation, atherosclerosis, sites also include sites where a transplant, including cells and/or organs will be placed within a mammal. In essence, generally any site within a mammal may be a suitable site. In particular, muscles, body cavities, particularly the abdominal or the peritoneal cavity are preferred sites.

"Vascularization" refers to the formation and maintenance of blood vessels. Stimulation or enhancement of vascularization is defined as increasing blood vessel formation and resulting blood circulation beyond that which would occur naturally.

The vascularization enhanced by the matrix is maintained in the organism. This is counter to the temporary vascular changes observed during an immune response. Inflammation is accompanied by proliferation of small blood vessels (angiogenesis). However, angiogenesis is often followed by regression or a loss of vessel structure. That is, the vessel integrity is not maintained following inflammation. In contrast, the vascularization or blood vessel formation of the invention results in mature vessels that maintain vessel integrity and survive as mature vessels. The process mimics vasculogenesis where a vascular network is established during embryogenesis. Thus, the vascularization of the invention is characterized by a network of mature blood vessels that is maintained in the host.

An effective amount of the matrix is applied to a site in a mammal where vascularization is desired. An effective amount is an amount necessary to stimulate the flow of blood to the desired anatomic site.

The methods of the invention can be used to increase vascularization in any mammal in need thereof. Mammals of interest include humans, dogs, cows, pigs, cats, sheep, horses, etc., particularly humans.

Any means may be used to apply or administer the matrix to the desired anatomic site. The amount of matrix applied will vary depending upon the amount of circulation needed, the weight and size of the recipient, the condition being treated, and the like. An effective amount of the matrix is an amount that promotes the desired amount of vascularization or blood flow and prevents an immune response and the formation of scar tissue.

As the matrix apparently stimulates vascularization by physical contact with tissue, the amount to be injected can be determined by (i) the linear length of tissue disruption to expose polar basement membrane sites and (ii) the volume of the disrupted tract or area to be filled with matrix.

The matrix may be used to increase vascularization in patients in need thereof. Thus, the methods of the invention are useful for enhancing wound healing, for decreasing scar tissue formation, i.e. following injury or surgery, and the like.

The matrix manufactured in accordance with the present invention is useful for the stimulation of new blood vessels without the presence of immune cells and the characteristic immune response. Thus, the use of the matrix of the invention results in vascularization without the formation of scar tissue. Therefore, the matrix may be utilized in any physiological setting where the formation of blood vessels is desired.

As indicated earlier, the matrix enables vascularization without stimulating immune cells. Thus, the matrix finds use in promoting wound healing. The matrix provides new blood vessel growth and fibroblasts to the site without the attraction of immune cells. The matrix prevents inflammation while promoting wound healing. Any tissue, or site, in need of repair or healing may benefit from application of the matrix to the site. Sites include those resulting from injury or surgery. The matrix may be applied to internal, or external surgical or injury sites to reduce the pain accompanying a classic inflammatory response, and to reduce scar tissue formation.

The matrix is also beneficial for superficial wound healing. Thus, it may be useful to apply to skin ulcers, burn areas, ulcers that form secondary to peripheral vascular disease, or other tissue damage.

The following examples are offered by way of illustration and not by way of limitation.

### EXPERIMENTAL

### Matrix Preparation

Place 835 ml of Medium 199 into a stirred beaker. While stirring, heat the solution to 50°C. Using a syringe, add 20 ml of albumin to the stirred solution. Pipette 63.28 µl of cysteine, 1 ml of L-glutamine and 200 µl of aminoguanidine into the stirred beaker. Add the following gamma irradiated dry raw materials: 120 grams of denatured collagen, 50 grams of dextran, and 0.1 grams of intact collagen. Use a glass stirring rod to aid mixing of the dry materials into solution. Pipette 8 ml of EDTA into the solution. Pipette 5 ml of L-glutamic acid, ml of L-lysine acetate, and 5 ml of arginine HCl into the stirred beaker. Note that the solution will turn yellow. Use 10% NaOH to adjust the pH of the matrix solution to a final pH of 7.40 + 0.05.

Cells may be embedded in the matrix of the present invention using the following procedure. Aspirate the supernatant from centrifuged cell pellets. Add a volume of cell culture medium and matrix to the cell pellets. Add a volume of matrix approximately equal to about 4 times the pellet volume. Add a volume of cell culture medium to the cell pellets equaling approximately 0.05 times the matrix volume added. Store the encapsulated cells at refrigerated temperatures if not using immediately.

### Example 1

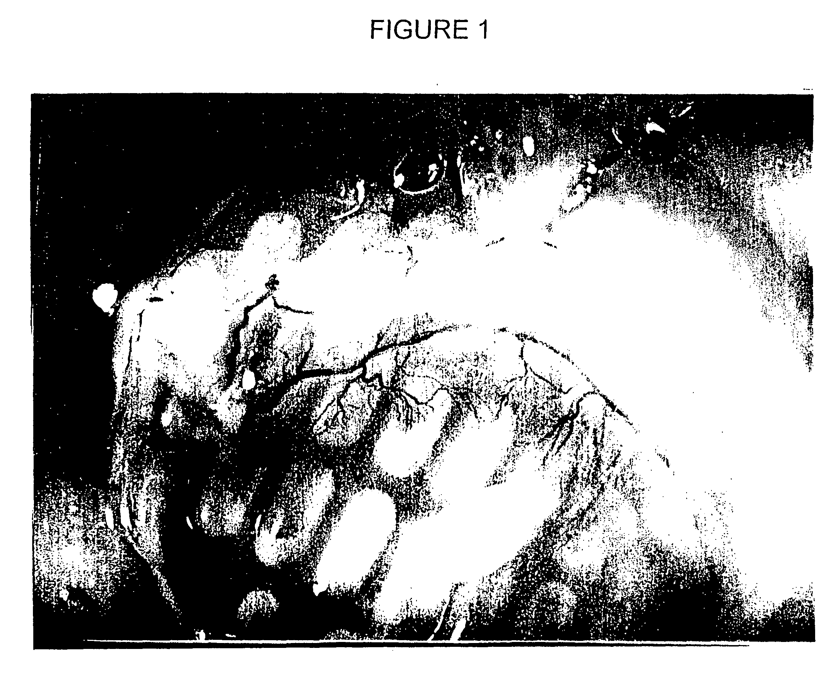

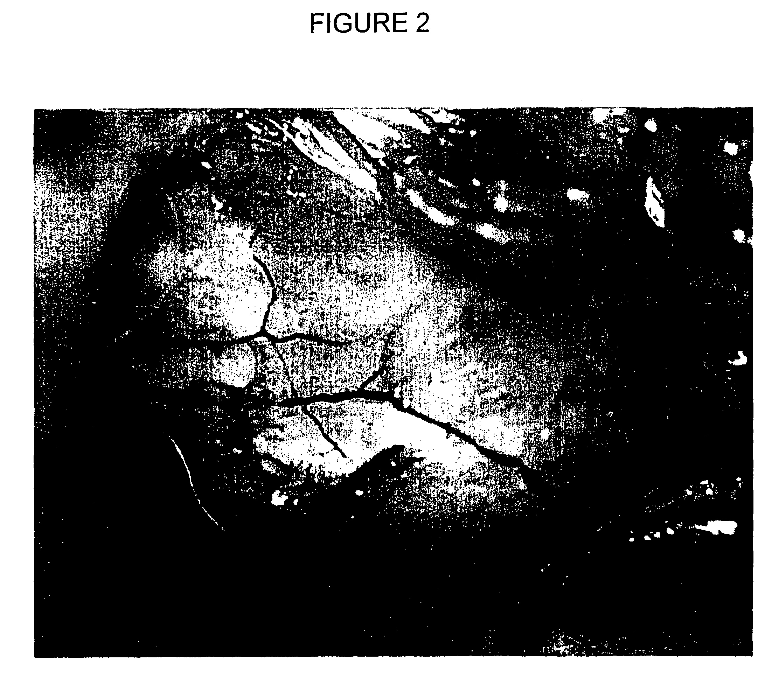

Normal 200-300 gram rats were injected intramuscularly with enhanced matrix. The animals were sacrificed at 4-6 days, and 21 days. Histologic sections revealed copious fibroblasts and new blood vessel formation at the injection site. Notably absent was the presence of immune or inflammatory cells. When placed around ENCELLIN XP devices, manufactured by Encelle, Inc., a thin fibrous capsule forms around the device which remains vascularized for the duration of the implant (out to four months in dogs and six months in rabbits). A non-adherent fibrous sheath with blood vessels was apparent at time of explant four months after surgical implantation in the dog. Figure 1 shows blood vessel formation 6 weeks post implant where a bioartificial pancreas that has a bioactive surface (tissue in matrix placed in wells covered by parylene N) was implanted between muscle layers with matrix liberally applied over the front and back. Figure 2 shows a vascularized device sheath 16 weeks post implant in a diabetic dog.

### Example 2

The ability of the matrix of the present invention to stimulate blood vessels inn a fibrous capsule was compared to matrigel with or without bFGF or VEGF when applied around polycarbonate devices intermuscularly in rats. Devices surrounded by these materials or no material were removed from some rats at 21 days and some at 50 days. bFGF and VEGF are two angiogenic growth factors currently in human clinical trials. Polycarbonate disks were implanted submuscularly in rats. The implants were removed after 21 and 50 days, stained with H&E, and Masson's Trichrome. The capsule thickness and vascular density of the capsule were evaluated.

| | |
|---|---|
| Abbreviation | Description |
| Uninvolved | Undisturbed tissue from the implant site |
| UPC | Uncoated polycarbonate disk |
| Coated | Polycarbonate disk coated with parylene |
| Matrigel | Parylene coated disk embedded in matrigel |
| VEGF | Parylene coated disk embedded in Matrigel + 3000 ng/ml VEGF |
| | (Vascular Endothelial Growth Factor) |
| BFGF | Parylene coated disk embedded in Matrigel + 3000 ng/ml bFGF |
| | (basic fibroblast growth factor) |
| EM | Parylene coated disk embedded in matrix of present invention |
| EM+ | Parylene coated disk embedded in matrix of present invention |
| | (including supplemental polar amino acids) |
| RS | Parylene coated disk embedded in matrix of present invention |
| | (including 10% rat serum) |
| RS+ | Parylene coated disk embedded in matrix of present invention |
| | (including supplemental polar amino acids and 10% rat serum) |

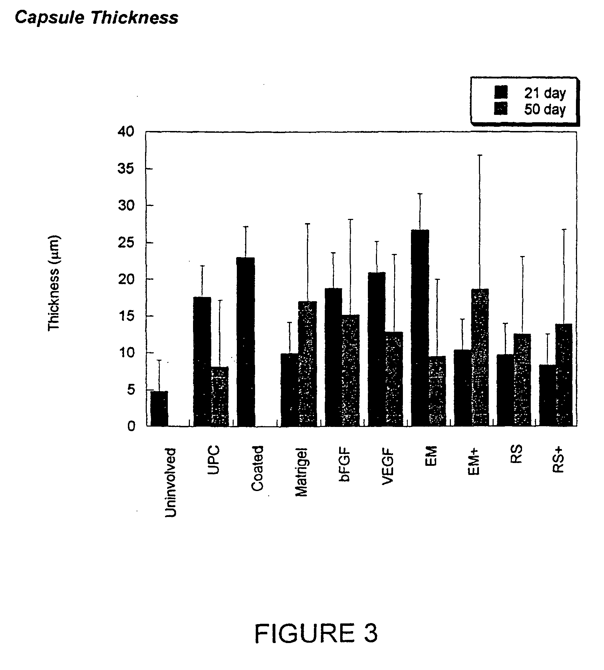

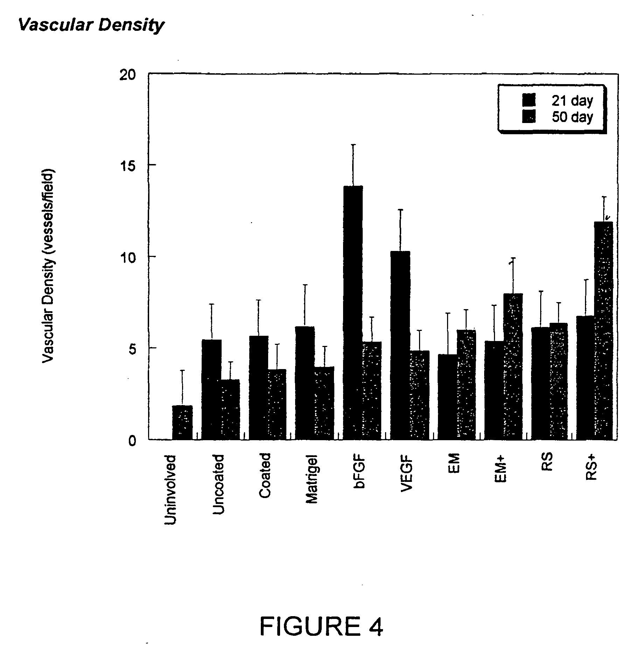

Only the matrix of the present invention stimulated new blood vessel growth between 21 and 50 days post injection. While all other groups stimulated initial new blood vessel growth up to 21 days, a diminution in both blood vessel number and fibrous capsule thickness was documented as mature scar tissue was formed. In addition, the matrix treated animals did not show the immune cell/inflammatory response observed in the bFGF and VEGF treated animals. See, Figures 3 and 4.

Figure 3 shows that after 21 days of implantation, the capsule thickness around the implanted devices were significantly (p<0.05) lower in the EM+, RS, and RS+ treated samples when compared to the coated polycarbonate disks (control). Disks coated with just Matrigel also showed a significantly (p<0.05) lower capsule thickness than the control. The presence of growth factors seems to eliminate any reduction in capsule thickness with pure Matrigel. After 50 days of implantation, no significant differences in capsule thickness with pure Matrigel. After 50 days of implantation, no significant differences in capsule thickness were observed in any treatment group.

Figure 4 shows that after 21 days of implantation, the observed vascular density surrounding the implanted disks as essentially the same for all treatments except for those with growth factors present. After 50-days of implantation, the additional vascular density observed with the growth-factor-enhanced Matrigel implants disappears. The vascular density of the implants with no matrix and the Matrigel covered implants decreased from 21 to 50 days. On the other hand, the vascular density provided by the matrices of the present invention (EM, EM+, RS, and RS+) all remained the same or increased.

The matrix can be applied to any area where new, physiologic vascularization is required. It can serve as an adjunct to provide vascularization to an implanted drug delivery or cell system.

The matrix might also prove useful in surgical applications where a minimization of scar tissue is desired. Because a thin fibrous capsule is formed that remains vascularized, the matrix may be applied to surgical breast implants to minimize painful adhesions.

All publications and patent applications mentioned in the specification are indicative of the level of those skilled in the art to which this invention pertains.

Although the foregoing invention has been described in some detail by way of illustration and example for purposes of clarity of understanding, it will be obvious that certain changes and modifications may be practiced within the scope of the appended claims.

## Claims

1. A use of a mixture of gelatin and dextran in the manufacture of a matrix for the treatment of a wound.

2. A use according to claim 1, wherein the matrix comprises 0.01 to 40 mM gelatin.

3. A use according to claim 1 or 2, wherein the gelatin comprises denatured collagen.

4. A use according to any one of claims 1 to 3, wherein the matrix further comprises an effective amount of a polar amino acid which is arginine, lysine, histidine, glutamic acid, or aspartic acid.

5. A use according to claim 4, wherein the effective amount of polar amino acid comprises 3 to 150 mM of polar amino acid.

6. A use according to claim 5, wherein the effective amount of polar amino acid comprises 10 to 65 mM of polar amino acid.

7. A use according to any one of claims 4 to 6, wherein the polar amino acid is selected from the group consisting of arginine, glutamic acid, lysine and mixtures thereof.

8. A use according to claim 7, wherein the matrix comprises:
2 to 60 mM of L-glutamic acid;
0.5 to 30 mM of L-lysine; and
1 to 40 mM of arginine.

9. A use according to claim 8, wherein the matrix comprises:
5 to 40 mM of L-glutamic acid;
1 to 15 mM of L-lysine; and
1 to 30 mM of arginine.

10. A use according to any one of the preceding claims, wherein the matrix further comprises 5 to 500 µM of L-cysteine.

11. A use according to claim 10, wherein the matrix comprises 15 to 25 µM of L-cysteine.

12. A use according to any one of the preceding claims, wherein the matrix further comprises 5 to 500 µM of a nitric oxide inhibitor.

13. A use according to claim 12, wherein the nitric oxide inhibitor comprises an L-arginine analogue.

14. A use according to claim 13, wherein the matrix comprises 15 to 25 µM of an L-arginine analogue.

15. A use according to claim 13 or 14, wherein the L-arginine analogue comprises aminoguanidine.

16. A use according to any one of the preceding claims, wherein the wound is a surgical wound.

17. A use according to any one of claims 1 to 15, wherein the wound is a superficial wound.

18. A use of a mixture of denatured collagen, dextran, aminoguanidine and an effective amount of a polar amino acid which is arginine, lysine, histidine, glutamic acid or aspartic acid in the manufacture of a matrix for the treatment of a wound.

19. A use according to claim 18, wherein the effective amount of polar amino acid comprises 3 to 150 mM of polar amino acid.

20. A use according to claim 18 or 19, wherein the polar amino acid is selected from the group consisting of arginine, glutamic acid, lysine and mixtures thereof.

21. A use according to claim 18, 19 or 20, wherein the matrix further comprises 5 to 500µM of L-cysteine.

## Patentansprüche

1. Verwendung eines Gemisches aus Gelatine und Dextran zur Herstellung einer Matrix zur Behandlung einer Wunde.

2. Verwendung nach Anspruch 1, worin die Matrix 0,01 bis 40 mM Gelatine umfasst.

3. Verwendung nach Anspruch 1 oder 2, worin die Gelatine denaturiertes Collagen umfasst.

4. Verwendung nach einem der Ansprüche 1 bis 3, worin die Matrix weiterhin eine wirksame Menge einer polaren Aminosäure umfasst, die Arginin, Lysin, Histidin, Glutaminsäure oder Aspartamsäure ist.

5. Verwendung nach Anspruch 4, worin die effektive Menge der polaren Aminosäure 3 bis 150 mM polare Aminosäure umfasst.

6. Verwendung nach Anspruch 5, worin die effektive Menge der polaren Aminosäure 10 bis 65 mM polare Aminosäure umfasst.

7. Verwendung nach einem der Ansprüche 4 bis 6, worin die polare Aminosäure ausgewählt ist aus der Gruppe, bestehend aus Arginin, Glutaminsäure, Lysin und Gemischen davon.

8. Verwendung nach Anspruch 7, worin die Matrix umfasst:
2 bis 60 mM L-Glutaminsäure;
0,5 bis 30 mM L-Lysin; und
1 bis 40 mM Arginin.

9. Verwendung nach Anspruch 8, worin die Matrix umfasst:
5 bis 40 mM L-Glutaminsäure;
1 bis 15 mM L-Lysin; und
1 bis 30 mM Arginin.

10. Verwendung nach einem der vorhergehenden Ansprüche, worin die Matrix weiterhin 5 bis 500 µM L-Cystein umfasst.

11. Verwendung nach Anspruch 10, worin die Matrix 15 bis 25 µM L-Cystein umafsst.

12. Verwendung nach einem der vorhergehenden Ansprüche, worin die Matrix weiterhin 5 bis 500 µM eines Stickstoffoxidinhibitors umfasst.

13. Verwendung nach Anspruch 12, worin der Stickstoffoxidinhibitor ein L-Arginin-Analoges umfasst.

14. Verwendung nach Anspruch 13, worin die Matrix 15 bis 25 µM eines L-Arginin-Analogen umfasst.

15. Verwendung nach Anspruch 13 oder 14, worin das L-Arginin-Analoge Aminoguanidin umfasst.

16. Verwendung nach einem der vorhergehenden Ansprüche, worin die Wunde eine Operationswunde ist.

17. Verwendung nach einem der Ansprüche 1 bis 15, worin die Wunde eine oberflächliche Wunde ist.

18. Verwendung eines Gemisches aus denaturiertem Collagen, Dextran, Aminoguanidin und einer wirksamen Menge einer polaren Aminosäure, die Arginin, Lysin, Histidin, Glutaminsäure oder Aspartamsäure ist, zur Herstellung einer Matrix zur Behandlung einer Wunde.

19. Verwendung nach Anspruch 18, worin die effektive Menge der polaren Aminosäure 3 bis 150 mM polare Aminosäure umfasst.

20. Verwendung nach Anspruch 18 oder 19, worin die polare Aminosäure aus der Gruppe, bestehend aus Arginin, Glutaminsäure, Lysin und Gemischen davon, ausgewählt ist.

21. Verwendung nach Anspruch 18, 19 oder 20, worin die Matrix weiterhin 5 bis 500 µM L-Cystein umfasst.

## Revendications

1. Utilisation d'un mélange de gélatine et de dextran dans la fabrication d'une matrice pour le traitement d'une blessure.

2. Utilisation selon la revendication 1, dans laquelle la matrice comprend 0,01 à 40 mM de gélatine.

3. Utilisation selon la revendication 1 ou 2, dans laquelle la gélatine comprend du collagène dénaturé.

4. Utilisation selon l'une quelconque des revendications 1 à 3, dans laquelle la matrice comprend en outre une quantité efficace d'un acide aminé polaire qui est l'arginine, lysine, histidine, acide glutamique ou acide aspartique.

5. Utilisation selon la revendication 4, dans laquelle la quantité efficace d'acide aminé polaire comprend 3 à 150 mM d'acide aminé polaire.

6. Utilisation selon la revendication 5, dans laquelle la quantité efficace d'acide aminé polaire comprend 10 à 65 mM d'acide aminé polaire.

7. Utilisation selon l'une quelconque des revendications 4 à 6, dans laquelle l'acide aminé polaire est choisi dans le groupe constitué de l'arginine, acide glutamique, lysine et des mélanges de ceux-ci.

8. Utilisation selon la revendication 7, dans laquelle la matrice comprend :
2 à 60 mM d'acide L-glutamique ;
0,5 à 30 mM de L-lysine ; et
1 à 40 mM d'arginine.

9. Utilisation selon la revendication 8, dans laquelle la matrice comprend :
5 à 40 mM d'acide L-glutamique ;
1 à 15 mM de L-lysine ; et
1 à 30 mM d'arginine.

10. Utilisation selon l'une quelconque des revendications précédentes, dans laquelle la matrice comprend en outre 5 à 500 µM de L-cystéine.

11. Utilisation selon la revendication 10, dans laquelle la matrice comprend 15 à 25 µM de L-cystéine.

12. Utilisation selon l'une quelconque des revendications précédentes, dans laquelle la matrice comprend en outre 5 à 500 µM d'inhibiteur d'oxyde nitrique.

13. Utilisation selon la revendication 12, dans laquelle l'inhibiteur d'oxyde nitrique comprend un analogue de L-arginine.

14. Utilisation selon la revendication 13, dans laquelle la matrice comprend 15 à 25 µM d'un analogue de L-arginine.

15. Utilisation selon la revendication 13 ou 14, dans laquelle l'analogue de L-arginine comprend de l'aminoguanidine.

16. Utilisation selon l'une quelconque des revendications précédentes, dans laquelle la blessure est une blessure chirurgicale.

17. Utilisation selon l'une quelconque des revendications 1 à 15, dans laquelle la blessure est une blessure superficielle.

18. Utilisation d'un mélange de collagène dénaturé, dextran, aminoguanidine et d'une quantité efficace d'un acide aminé polaire qui est l'arginine, lysine, histidine, acide glutamique ou acide aspartique dans la fabrication d'une matrice pour le traitement d'une blessure.

19. Utilisation selon la revendication 18, dans laquelle la quantité efficace d'acide aminé polaire comprend 3 à 150 mM d'acide aminé polaire.

20. Utilisation selon la revendication 18 ou 19, dans laquelle l'acide aminé polaire est choisi dans le groupe constitué de l'arginine, acide glutamique, lysine et des mélanges de ceux-ci.

21. Utilisation selon la revendication 18, 19 ou 20, dans laquelle la matrice comprend en outre 5 à 500 µM de L-cystéine.
